# EUROPEAN PATENT APPLICATION

(11) **EP 4 691 515 A1**
(43) Date of publication of application: **11.02.2026**
(21) Application number: 24193886.9
(22) Date of filing: 09.08.2024
(51) Int. Cl.: A61M 5/14, A61M 5/162, A61M 5/142, A61M 5/145, A61M 5/158, A61M 5/315

(54) **A MODULAR PATCH INJECTION SYSTEM**

(71) Applicant: Ypsomed AG, 3401 Burgdorf (CH)
(72) Inventor: Scheurer, Simon, 3006 Bern (CH); Pirker, Gerhard, 4500 Solothurn (CH)
(74) Representative: Meier Obertüfer, Jürg

(57) **Abstract**

A novel modular patch injection system is disclosed. The system comprises a reusable drive module which can be connected to a first and a second disposable reservoir module, the reservoir module being adapted to a certain volume of the reservoir contained.

## Description

### FIELD OF THE INVENTION

The present invention relates to the field of medical devices, specifically to on-body delivery systems or wearable patch injection devices, adhesively attachable to a patient's skin and designed for subcutaneous administration of therapeutic agents. More particularly, this invention pertains to a modular injection system that offers flexibility, ease of use, and enhanced comfort for patients requiring regular administration of large and possibly non-uniform volumes of medication injections.

### BACKGROUND OF THE INVENTION

The administration of therapeutic agents via injection is a common practice for the management of various chronic conditions such as diabetes, autoimmune disorders, hormonal deficiencies and, increasingly, obesity. Traditional injection methods, including syringes, autoinjectors and pen injectors, are suitable for small injection volumes of up to 5 ml and relatively short injection times. However, higher injection volumes reduce the required frequency of injections and can possibly help to establish subcutaneous delivery for new therapeutic areas and drug modalities that require larger single-dose volumes. Some of these therapeutic agents require volumes of 10 ml or more, leading to injection times of several minutes and rendering handheld devices impractical. Wearable injection devices have emerged as a solution to this issue, allowing for continuous or on-demand drug delivery with minimal patient intervention and improved comfort.

Existing wearable patch injection devices, such as the ones disclosed in EP4114483A1 or EP4247457A1 are often designed as disposable devices, intended for a single injection and are to be disposed after use. This results in a large ecological footprint and a waste of parts which could be used more than once. To overcome this problem, semi-reusable devices such as the one described in WO24052442A1 have been developed. These comprise a reusable part, and a disposable part containing the needle and the medicament reservoir, which ensures sterile conditions for each delivery event.

Existing devices are, however, usually adapted for a single therapeutic agent and a given reservoir size, which means that expensive modifications to both the reusable part and the disposable part of the device are required, if a reservoir of a volume other than the designated size is needed.

### DESCRIPTION OF THE INVENTION

It is an objective of the present invention to overcome the mentioned limitations of the prior art and to provide a modular patch injection system, adhesively attachable to a patient's body, that can accommodate various reservoir sizes. This objective is solved by the invention as defined in the independent claim of the present application; specific variants are disclosed in the dependent claims and particularized in the description.

The invention addresses the limitations of current injection devices by introducing a semi-reusable wearable patch injection system that is modular and combines patient comfort, user-friendliness, and flexibility in terms of reservoir size. The system is designed to be worn on the body, providing a convenient method for the regular administration of medication. Its modular architecture allows for customizable components that can be tailored to different types of medications, volumes, and delivery schedules. The semi-reusable system comprises a reusable drive unit and disposable reservoir units releasably attachable to the drive unit by a single user or patient, or even a group of individuals, thereby avoiding at least disposal of a drive unit after each medication delivery event. The system comprises interchangeable reservoir units alternatively or subsequently attachable to a single, unique, reusable drive unit, and enclosing drug reservoirs of distinct size or volume. With such a system, the user or patient is not only enabled to re-use the drive unit, but to use the drive unit to inject different volumes of medication. Shared or concurrent use of the drive unit, within a family of patients or by a health-care professional, for the same or for plural indications is thus enabled.

Each reservoir unit has a housing with a shape adapted to a reservoir size of the reservoir enclosed, leading to injection devices (with drive and reservoir unit attached to each other) having a distinct appearance respective of the size of the reservoir. In the present context, the shape of a housing is defined by the geometry of the housing and/or its size, such that two reservoir unit housings which differ exclusively in one dimension, e.g. a length or diameter, qualify as different or distinct. Distinct shapes of the reservoir units require distinct molding tools (if manufactured by injection molding) but minimize material employment and waste due to avoidance of dead space in an unnecessary large reservoir unit. Such waste, or corresponding savings in material, is more pronounced for larger differences in reservoir size, in particular for a difference of 5 ml or more. This leads to reduced cost of manufacturing and a reduced environmental footprint as compared to a system with single reservoir unit housing and adapters to account for distinct reservoirs.

Key features include a compact form factor, ease of attachment and detachment, and a high degree of modularity.

In summary, this invention represents a significant advancement in the field of wearable medical devices, providing a versatile and user-friendly solution for patients requiring regular large volume injections. By addressing the challenges associated with traditional injection methods and existing wearable devices for large injection volumes, the wearable modular injection system aims to improve practicality and easy of manufacturing.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula. "Left" and "right" are used to refer to the left and right side respectively, when viewing from the proximal end in distal direction. "Bottom" or "bottom side" are used to refer to the side or surface of the device or system which is attached or pressed onto the skin of a patient or user, wherein "top" or "top side" are used to refer to the side or surface facing the opposite direction.

The term "injection system" or "injector" refers to a device that is removed from the injection site after each medication event or drug delivery process, whereas the term "infusion system" refers to a device with a cannula or needle that remains in the skin of the patient for a prolonged period, for example, several hours.

According to a first aspect of the disclosure, a system for dispensing, i.e. injecting, a liquid medicament is provided, parts of which can be assembled or attached to each other to form an injection device which can be attached to a user's skin via an adhesive surface. The system is a modular injection device, comprising a reusable drive module and at least a first and a second disposable reservoir module which can interchangeably and releasably be attached to said drive module. The reservoir modules are adapted to receive a medicament reservoir such as a cartridge or a pre-filled syringe. The reusable drive module contains an energy source such as a compression or torsion spring or a battery, and a drive mechanism adapted to drive a plunger rod into the reservoir during injection.

The plunger rod preferably is a segmented plunger rod with separate discrete elements or segments, that are connected via hinges, pivots or other flexible means of connection. This allows for a space-saving arrangement of the drive train and the plunger rod, as the drive train and the plunger rod need not be in a straight line but can be arranged in a more space-efficient manner. The plunger rod or part of the plunger rod may be in threaded engagement with a positionally fixed threaded rod or shaft whose rotation causes the plunger rod to be displaced and pushed into the reservoir.

The drive unit comprises an interface to transport energy and/or a mechanical force or torque from the drive unit to the disposable reservoir unit. The energy may be used to drive an injection needle, housed in the disposable reservoir unit, into the skin of a user, thus establishing a fluid path between the medicament reservoir and the injection needle. The fluid path may be established by piercing a septum and/or a stopper on an end of the medicament reservoir. Upon insertion of the needle, the device may be ready to dispense medicament from the reservoir into the body of a patient. The drive unit may comprise a button or other means to initiate insertion of the needle, and/or the injection and a visual indication means to indicate a status of the device, such as a screen or an LED progress bar.

According to an aspect of the invention, the system comprises a first and a second disposable reservoir module, wherein the reusable drive module can be connected to the first or the second reservoir module to form an injection device for the subcutaneous delivery of a medicament trough an injection needle in the disposable reservoir unit. The second reservoir unit has, at least in parts, a different geometry and a different shape than the first reservoir unit.

The first and second reservoir module are shaped in a way to allow attachment to a patient's body and may comprise an adhesive surface which is planar or may be curved or bent according to the region of the body where it is supposed to be attached to. Alternatively, the adhesive surface may be part of the drive module and may comprise a replaceable adhesive layer that can be replaced after each use. Further, both the drive module and the first and second reservoir module may comprise adhesive surfaces. In any case, the adhesiveness may be such that the weight of the entire device is supported throughout the injection and that the device may be removed without pain by the patient after the injection is finished. The adhesive surface may be covered by a protective layer or sheet that the patient must remove before attaching the device to the body.

The first and second reservoir module may be configured to receive and immovably hold in place a medicament reservoir. The medicament reservoir may be a cartridge, an ampoule, a pre-filled syringe or any other type of suitable container. The medicament reservoir may preferably be of cylindrical shape with a longitudinal axis perpendicular to the cylinder bottom and may be tapered towards one end of said longitudinal axis. The tapered end may be sealed by a plastic or metal crimp and/or a septum which can be pierced to establish a fluid path to the injection needle. The opposite end may be closed by a bung or plunger inside the reservoir that forms a tight seal or a sealing gap with the reservoir walls and may be displaced towards the tapered end by the plunger rod to dispense medicament through the injection needle, when the fluid path is established.

The first reservoir unit may be configured to accommodate a medicament reservoir of a first size, particularly, a medicament reservoir of a first cylinder diameter and the second reservoir unit may be configured to accommodate a medicament reservoir of a second size, particularly, a medicament reservoir of a second cylinder diameter, wherein the second diameter is preferably greater that the first diameter. Parts or sections of the second reservoir unit are adapted respective to said difference in size or diameter and are of different shape and size when compared to the first reservoir unit or have a different relative orientation to the drive unit when attached thereto. In particular, the adhesive surface of the second reservoir module may have a different rotational orientation or a difference in a distance relative to parts of the drive module such as a cam or a shaft of the drive mechanism. Possible reservoir volumes or sizes include at least a 10ml and a 20ml reservoir or cartridge.

The adhesive surface on the first and the second reservoir unit may have the form of a sticky coating or layer which serves as bonding between the reservoir unit and the user's skin. Alternatively, and preferably, the adhesive surface on the first and the second reservoir unit may be supplied as a patch which essentially has the shape of a sheet with a first upper surface, adhesively attached to the bottom of said module and a second bottom surface which can be attached to the body of the user. The bottom surface for attaching to the user's body may initially, in a delivery state, be covered by a cover sheet or release liner that maintains adhesiveness and sterility of the patch and forms a patch unit together with the patch itself. The patch unit, specifically the size and outline of the patch unit for the first and second reservoir unit may be adapted or adjusted to the shape of the respective reservoir unit or, preferably a single, universal patch unit is supplied which can be used for both the first and the second reservoir unit, such that the first and the second reservoir unit have identical patch units.

The needle insertion mechanism for inserting the injection needle into the skin of the patient and for inserting the reservoir needle into the cartridge, is preferably contained in an inner housing affixed to an inner surface of the housing of the first and/or second disposable unit by means of a snap fit connection, an adhesive connection or any other suitable means of fixation. The inner housing surrounding the needle insertion mechanism allows to keep the needles and the mechanism, including the fluid path between the injection needle and the reservoir needle, sterile by creating a surrounding barrier. The mechanism for inserting the needles together with the injection needle and the housing thus form a sterile needle unit, which can be assembled under sterile conditions at a different location than the rest of the system. The needle unit comprises a reservoir needle which can protrude out of the needle unit in a direction parallel to the cartridge and an injection needle which can protrude out of the needle unit in an injection direction, wherein the two directions are preferably perpendicular. The needle unit is preferably identical for both the first and the second reservoir unit and has a shape such that it can fit in both the first and the second reservoir unit, which decreases cost and increases ease of manufacture.

The drive unit comprises a drive mechanism to supply a mechanical force or torque to other parts of the system. The drive mechanism for driving the plunger rod, the needle insertion mechanism and/or other parts of the system may be purely mechanical and comprise springs, gears shafts and/or other mechanical parts known in the art. The mechanism may preferably comprise an electric motor for driving the plunger rod and/or the needle insertion mechanism when the drive unit is attached to the first or the second reservoir unit. The electric motor is adapted to convert electrical energy supplied to it into mechanical energy required by other parts of the system. The electric motor may be an induction motor, synchronous motor, direct current (DC) motor or a special-purpose motor such as a stepper motor, preferably a hybrid stepper motor, or a servo motor. Using an electric motor instead of a mechanical drive mechanism allows for precise control of the injection parameters and for easy incorporation of sensor feedback into the motor control. The motor control unit is adapted to control the motor on/off state, speed, gearing ratio and other parameters. The motor control unit may comprise an integrated circuit which may be integrated into a printed circuit board (PCB), a processor, a memory unit, and other electronic components for controlling the electric motor. The electric motor may be supplied with energy by a battery connected thereto.

The battery may be integrated into the housing of the drive module through a secure battery compartment or a sealed battery enclosure, providing easy access for battery replacement or recharging, or may be attached thereto via an interface that allows the flow of energy from the battery to the drive module. The battery may be a disposable battery such as an alkaline battery or a rechargeable battery such as a nickel-cadmium or a lithium-ion battery, with a capacity sufficient to power the device for multiple injection cycles. In case of a rechargeable battery, the drive unit may comprise a recharge port, such as an USB-C port for connecting a current source and recharging the battery. The battery may be connected to or equipped with a battery management system to monitor and control various operational parameters, including but not limited to, charge and discharge rates, temperature, and voltage levels.

The battery may be connected to an electric motor which produces the mechanical force for driving a plunger rod located in the drive module. The drive unit may contain additional electronic components for controlling the motor and providing human interface in the form of status signals or input/output elements to allow a patient to interact with the device. The plunger rod may be a solid or hollow cylinder that is adapted to bias a bung in a medicament reservoir immovably held in the disposable module, such as to cause medicament to flow from the reservoir into a user's body via a needle penetrating the skin. If the energy source is provided in the form of a spring, the spring might be adapted to directly bias the plunger rod or to exert force on the plunger rod via a mechanical transmission.

When the drive unit is attached to the first or the second reservoir unit, the user may initiate the injection, preferably by pressing a button or by actuating another interface means provided. Upon actuation the system is configured to continuously dispense medicament from the reservoir through the injection needle until a maximal injection dose is reached or until the reservoir is empty. In particular, the injection process may not be an interrupted sequence of dispensing events such as in an automatic insulin infusion system. The injection time is preferably not longer than 60 minutes, more preferably not longer than 60 minutes, after which the device may be detached from the user's skin and the administration procedure may be finished.

In one embodiment of the system, the first and/or second reservoir unit are adapted to receive an adapter to fit reservoirs of a smaller diameter into the space designed for reservoir with a relatively larger diameter. The adapter may have an outer diameter corresponding to the diameter of a larger reservoir and a space or bore that immovably holds a smaller reservoir, having an inner diameter corresponding to the diameter of a smaller reservoir. The adapter may preferably be fixed to the first or second reservoir unit by means of a snap connection, threaded connection or other suitable means. The adapter may also be held in place exclusively by friction.

In a preferred embodiment, the second reservoir unit is adapted to accommodate reservoirs of a larger diameter and/or size than the first reservoir unit. To accommodate reservoirs of a larger diameter, the device geometry and/or shape must be adjusted accordingly. The space for receiving the reservoir may be larger or wider in the second reservoir unit than in the first reservoir unit. A lid or holder fixing the cartridge and comprising a viewing window granting visual access to the cartridge may be shifted outwards relative to the housing and may be of a larger size. In a preferred embodiment the bottom of the housing of the second reservoir unit may be slanted or tilted relative to the bottom or base of the drive unit or relative to other parts of the drive unit such as the threaded rod, specifically the longitudinal axis of the threaded rod, such that a rotational orientation of the bottom surface and the attached patch unit relative to said longitudinal axis, may be different for the first and the second reservoir unit. In this embodiment, the injection needle of the second reservoir unit, which will protrude the bottom of the reservoir unit during injection, may be angled or tilted relative to the patch unit, because the needle unit will not be tilted with the bottom. In an alternative embodiment the needle unit may have the same rotational orientation as the bottom.

In the second reservoir unit comprising the tilted or slanted patch unit, a distance from the threaded rod to the patch unit, preferably measured in a direction perpendicular to the longitudinal axis of the threaded rod, may be different, i.e. greater, than the corresponding distance in the first reservoir unit.

The reusable drive unit comprises an engagement means for releasable attachment of the drive unit to the disposable unit by engaging a counter engagement means on the reservoir unit. The engagement means may comprise hooks or ledges to engage a stopping surface on the disposable unit or the engagement means might comprise a stopping surface to be engaged by hooks or ledges on the disposable unit. The first engagement means may comprise a male or a female part of a bayonet connection, adapted to engage the corresponding male or female counterpart on the disposable unit.

In a preferred embodiment, the drive unit and the first and second reservoir unit are adapted to be attached and released from each other in a linear way, such that the relative motion for engagement is one-dimensional. This may be achieved by guiding means such as channels on one part and protrusions on the other, or any other parts on one unit that can form a positive or form fit with the respective other unit along the direction of engagement such that the unit are guided towards each other in a linear fashion when attaching the first or second reservoir unit to the drive unit.

### BRIEF DESCRIPTION OF THE DRAWINGS

The subject matter of the invention will be explained in more detail in the following text with reference to preferred exemplary embodiments which are illustrated in the attached drawings, in which:
- Fig. 1: Left side: Drive unit 1 attached to first reservoir unit 2; perspective view Right side: Drive unit 1 attached to second reservoir unit 2'; perspective view
- Fig.2: Left side: Drive unit 1 attached to first reservoir unit 2; proximal view Right side: Drive unit 1 attached to second reservoir unit 2'; proximal view
- Fig.3: Perspective view of drive unit 1
- Fig.4: Top: Lateral view of drive unit 1 Bottom: Section view of drive unit 1 along A-A plane
- Fig.5: Perspective view of second reservoir unit 2'
- Fig.6: Top: Section view of drive unit attached to second reservoir unit 2' Bottom: Section view of drive unit attached to first reservoir unit 2

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

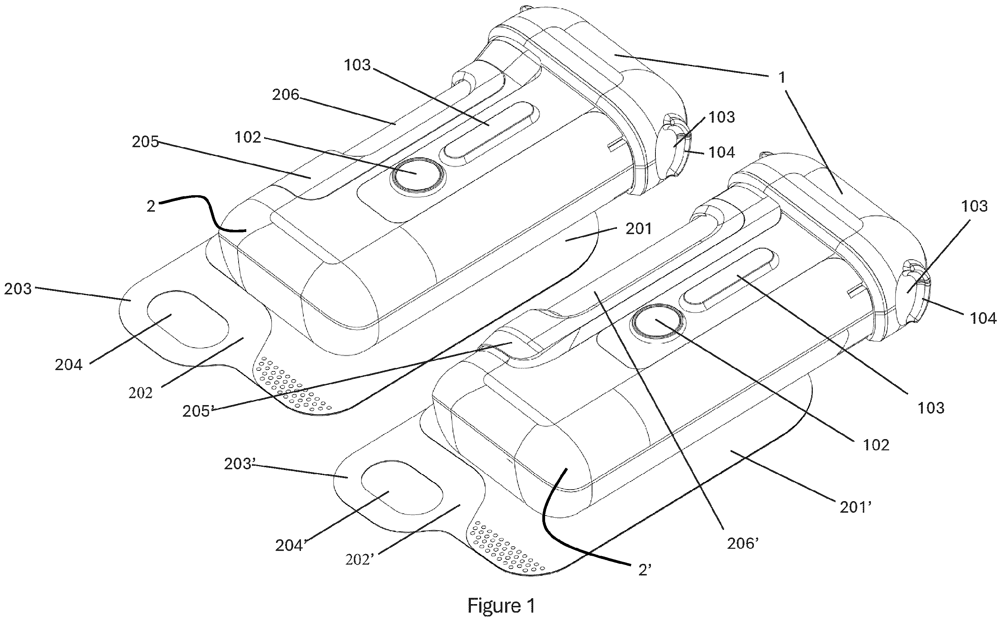

The left side of Figure 1 shows a perspective view of an embodiment of an injection system according to the present invention. Depicted is the drive unit 1 attached to the first reservoir unit 2. The first reservoir unit is adapted to be attachable to a user's skin via planar patch 201, which comprises an adhesive surface. The adhesive surface is initially covered by release liner 202 to protect the adhesive bottom surface from dirt and/or other contaminants that could impair adhesiveness and/or sterility. The patch 201 preferably has a surface area of at least the same size as the bottom surface area of the disposable unit 2. The release liner 202 preferably has a surface area of at least the size of the patch 201 and comprises at least one area or section where it protrudes the outer boundary of patch, forming a pull tab 203 that can be gripped by the user, allowing the user to remove the release liner and attach the device to the body. The pull tab 203 may comprise an aperture 204 or a roughened surface or any other means to facilitate gripping and pulling the release liner for the user. The release liner may be attached to seals or membranes on the inside of the reservoir unit (such as Tyvek membranes) forming sterile barriers that protect the fluid path between injection needle and reservoir from contamination during storage, such that upon removal of the release liner the seals or membranes are removed as well. The patch 201 may comprise skin sensors such as capacitive sensors which are connected to the electronics of the motor control unit such that the injection might only be initiated if the injection device is attached to the skin of a user. The reservoir unit may comprise a reservoir holder 205, which is preferably made of the same material as the housing of the reservoir unit and can be snapped or otherwise fixed onto said housing after insertion of the medicament reservoir 3. The reservoir holder 205 may comprise an aperture that forms a viewing window 206, allowing the user visible access to the reservoir and visual tracking of injection progress as the bung 31 in the reservoir moves in distal direction.

The right side of Figure 1 shows the same drive unit 1 being attached to the second reservoir unit 2'. The housing of reservoir unit 2' is longer along the direction of the reservoir when compared to the first reservoir unit 2 and has different dimensions which are adapted to the outer dimensions of a second, larger medicament reservoir 3'. The second reservoir unit 2' may comprise patch 201', release liner 202' and pull tab 203' with aperture 204' all of which have the same functionality as their respective counterparts of the first reservoir unit but may be of different shapes and geometries and are preferably of larger size. The second reservoir unit 2' may comprise a reservoir holder 205' which is shifted outwards when compared to reservoir holder 205 and comprises a viewing window 206'.

The drive unit 1 comprises a button 102 that allows the user to initiate the injection and may comprise additional functionality such as causing the device to display information like battery percentage or number of performed injections via signalling element 103, which has the shape of an elongated protrusion and might be a display or an arrangement of signalling elements such as light emitting diodes (LEDs). On a lateral surface, gripping portions 104 are formed on each side of the drive unit, which are proximally bounded by gripping protrusions 105 allowing the user to grip the drive unit and disengage the attachment between the drive unit 1 and the reservoir unit 2, as will be described in detail later.

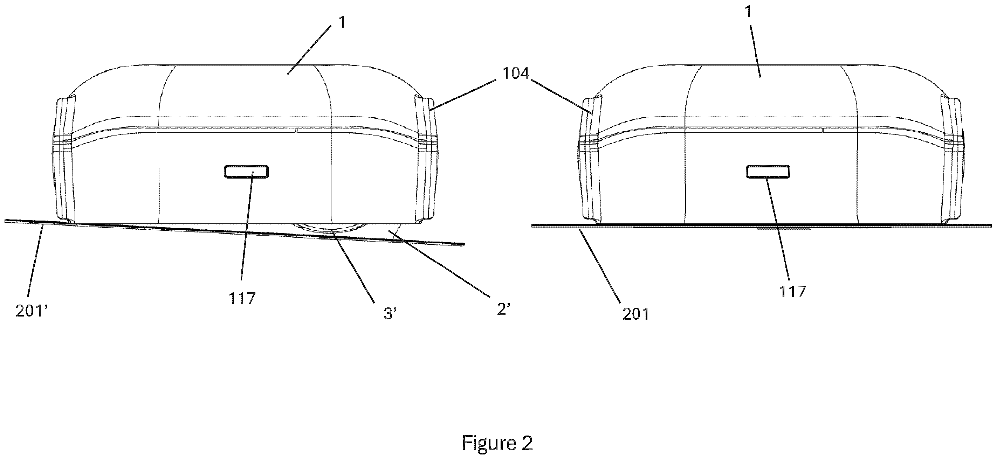

The left side of Figure 2 shows a proximal view of the drive unit 1 attached to the second reservoir unit 2', wherein the right side shows drive unit 1 attached to the first reservoir unit 2. Visible on each side are the gripping protrusions 105 of drive unit 1. According to an aspect of the present invention, the adhesive surface of the second reservoir unit 2' may have a different rotational orientation relative to the drive unit 1 when compared to the adhesive surface of the first reservoir unit 2, which is represented by the slanted patch unit 201', 202'. This allows to accommodate a medicament reservoir of a larger diameter in the second reservoir unit 2' whilst keeping material usage to a minimum.

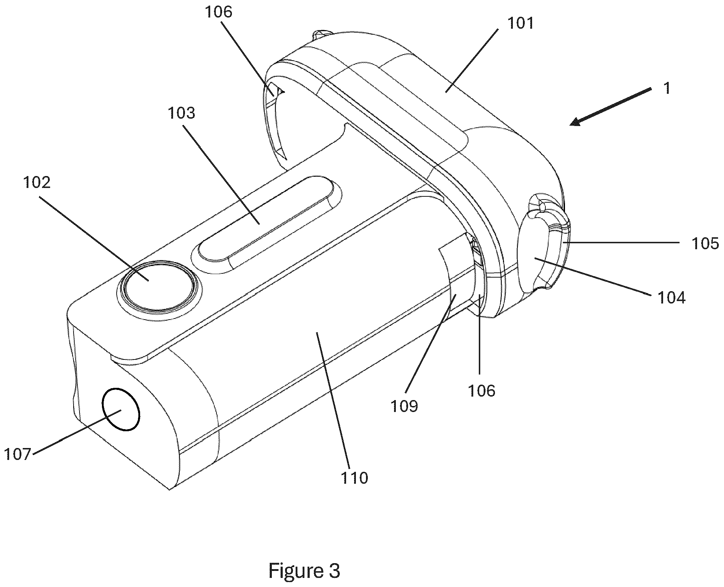

Figure 3 shows a perspective view of an embodiment of the drive unit 1. The drive unit is enclosed by housing 101, which comprises a slightly thicker proximal part and an elongated distal part comprising injection button 102 and signalling means 103. On the proximal end, gripping portions 104 with gripping protrusions 105 are formed. On the rim of the distally oriented surface of the proximal part, two engagement recesses 106 are formed, which provide access for the counter engagement means 210, 210' of the first and second reservoir unit 2,2'. The elongated distal part of drive unit 1 is shifted to the left side to leave room for the medicament reservoir 3 or 3', contained in the reservoir unit. On the right side of the elongated distal part, a cut-out section 110 provides access for the lateral convex guide wall 211, 211' of the reservoir unit 2 or 2', which helps to linearly guide the drive and the reservoir unit when connecting the two. On the distal end surface of the distal part of drive unit 1, a mechanical drive interface 107 is formed as a hole in the housing 101, which receives the cam shaft 214, 214' of the first or second reservoir unit 2,2'. The mechanical drive interface might also have any other suitable form of shape for transmitting a force or torque from the drive unit 1 to the first or second reservoir unit 2 or 2'.

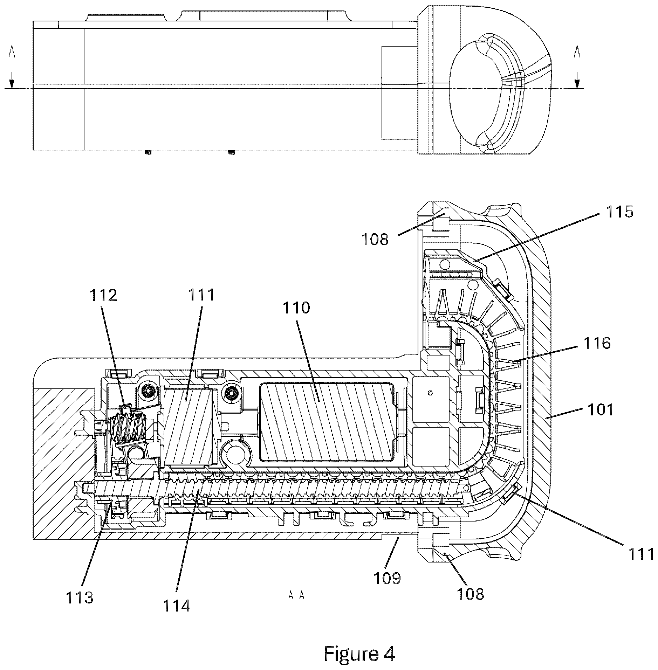

The top of Figure 4 shows a lateral view of the drive unit 1, wherein the bottom shows a section view of drive unit 1, cut along the plane A-A. On the proximal part of the housing 101, engagement means 108 are formed, which take the form of approximately triangular recesses, that can engage counter engagement means 210, 210' of the first and second reservoir unit. To attach the drive unit 1 to the first or second reservoir unit, the user inserts the distal part of the drive unit 1 into the space between the convex guide wall 211, 211' and the reservoir holding part of the reservoir unit and pushes in distal direction. The drive unit is contained and linearly guided in the space between the convex guide wall 211,211' and the inner side wall of the reservoir holding part of the reservoir unit. When the counter engagement means 210, 210' of the reservoir unit reach the distal end wall of the proximal part of the drive unit, they can flex inwards as the chamfered edges 210a, 210'a about against the wall of the housing 101, until they snap radially outwards into the recesses 108 forming a releasable snap connection. On the left side of the drive unit, this flexing motion is enabled by a cut-out 109. To release the attachment between drive unit 1 and reservoir unit 2,2', the user grips the drive unit 1 by the gripping portions 104 and presses inwards, causing the counter engagement means 210, 210' to flex inwards and disengage from the recesses 108, upon which the two units can be pulled apart.

The drive mechanics are contained in a drive housing 115, which forms the drive carrier, and which may be glued, snapped, or otherwise fixed in the drive unit housing 101. The drive carrier is closed by a lid (not shown) via snap elements 111. This allows for separate assembly of the drive mechanics and facilitates transportation in case the final assembly is done at a different location. A battery 110 provides electrical energy for the stepper motor 111, which drives gear shaft 112. Gear shaft 112 is in torque-transmitting engagement with threaded rod 114, which is in torque-transmitting engagement with ratchet wheel 113 when rotating in one direction but not in the other. Ratchet wheel 113 can engage the first or second mechanical interface (cam shaft 214, 214') of the reservoir unit 2 in a torque-transmitting way when drive unit 1 is attached to the first or second reservoir unit 2, 2'. When the user presses injection button 102, stepper motor 111 rotates in a first direction, which will result in the ratchet wheel 113 rotating and transmitting torque to the first or second mechanical interface 214, 214' causing the injection needle to be inserted into the skin of the user. The stepper motor then changes direction of rotation resulting in the outer thread of threaded rod 114 rotating in the inner thread of segmented plunger rod 116, causing the plunger rod to move in a dispensing direction and plunger rod flange 118 to press against bung 31,31' resulting in dispensing of medicament from the reservoir.

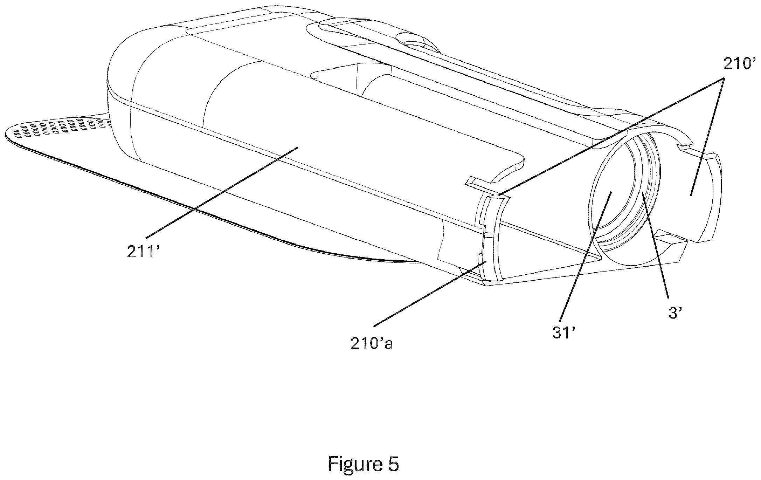

In Figure 5, a proximal perspective view of the second reservoir unit 2' is shown. On the left side, convex guide wall 211' is formed, which helps the user to guide the relative alignment between drive unit 1 and second reservoir unit 2' during attachment. At the proximal end, on both the left and right side, two cantilever snap arms witch chamfered edges 210'a are formed, representing counter engagement means 210'. On the right side, a chamber provides access for the medicament reservoir 3', which is confined by a lateral side wall on the left and by reservoir holder 205' with viewing window 206' on the right side.

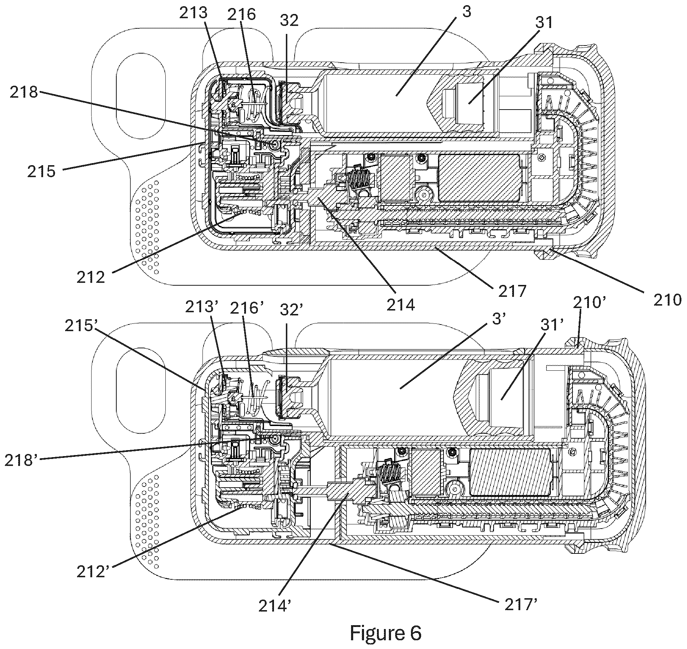

Figure 6 shows a section view of the drive unit 1 attached to first reservoir unit 2 on the top and a section view of the drive unit 1 attached to the second reservoir unit 2' on the bottom. Referring first to the drawing on the top, drive unit 1 is attached to the first reservoir unit 2 via engagement means 108 and counter engagement means 210. Cam shaft 214 is in engagement with ratchet wheel 113 of drive unit 1. The entire needle insertion mechanism is contained in the inner needle unit housing 215 forming one needle unit, which is additionally sealed by sterile barriers at the openings for the reservoir needle and for the injection needle. The sterile barriers are then connected to and removed with release liner when the system is attached to a user's body. This allows for manufacturing of the needle unit under sterile conditions, wherein the final assembly and mounting in reservoir unit 2 may be performed at a different location and wherein the shape of reservoir unit 2 might be adapted to e.g. different cartridge sizes without changing the needle unit. When the user initiates the injection, cam shaft 214 starts to rotate, causing the skin needle spring to partially relax and insert the skin needle (not shown), which is in fluid connection with the reservoir needle 216. At the same time, reservoir needle spring 213, causes reservoir needle 216 to penetrate stopper 32, which seals the reservoir 3 distally. A fluid path is thus established between the reservoir and the injection site. Details of the needle insertion mechanism have previously been disclosed in WO2021/156130Al and are thus omitted here.

The bottom part of Figure 6 shows drive unit 1 connected to second reservoir unit 2'. Although dimensions of outer reservoir unit housing 217' may be different for the second reservoir unit 2', when compared to first reservoir unit 2, the same needle unit can be used for both, except for cam shaft 214' which must be longer than cam shaft 214 to account for the difference in length between reservoir 3 and reservoir 3'.

**LIST OF DESIGNATIONS**

| | | | |
|---|---|---|---|
| 1 | Drive Unit | 210, 210' | Counter Engagement Means |
| 101 | Drive Unit Housing | 211, 211' | Convex Guide Wall |
| 102 | Injection Button | 213, 213' | Reservoir Needle Spring |
| 103 | Signaling Means | 214, 214' | Cam Shaft |
| 104 | Gripping Portions | 215, 215' | Needle Unit Housing |
| 105 | Gripping Protrusions | 216, 216' | Reservoir Needle |
| 106 | Engagement Recesses | 217, 217' | Reservoir Unit Housing |
| 107 | Mechanical Drive Interface | 218, 218' | Injection Needle |
| 108 | Engagement Means | 3, 3' | Medicament Reservoir |
| 109 | Cut-Out | 31, 31' | Medicament Reservoir Bung |
| 110 | Battery | 32, 32' | Stopper |
| 111 | Stepper Motor | | |
| 112 | Gear Shaft | | |
| 113 | Ratchet Wheel | | |
| 114 | Threaded Rod | | |
| 115 | Drive Housing | | |
| 116 | Segmented Plunger Rod | | |
| 117 | Charging Port | | |
| 118 | Plunger Rod Flange | | |
| 2, 2' | Reservoir Unit | | |
| 201, 201' | Patch | | |
| 202, 202' | Release Liner | | |
| 203, 203' | Pull Tab | | |
| 204, 204' | Aperture | | |
| 205, 205' | Reservoir Holder | | |
| 206, 206' | Viewing Window | | |

## Claims

1. A system for administering a liquid drug from a reservoir (3, 3'), comprising a reusable drive unit (1) and a first and a second reservoir unit (2, 2'),
the drive unit comprising a plunger rod (116), adapted to displace a bung (31, 31') in the reservoir, an energy storage (110), an engagement means (108) and a mechanical drive interface (107, 113),
the first reservoir unit comprising;
a) a first housing (217),
b) a first patch unit (201, 202) for attaching to a patient's body during administration,
c) a first counter engagement means (210) for releasable attachment of the drive unit to the first reservoir unit via the engagement means and the first counter engagement means and,
d) a first mechanical interface (214) connected to a first needle insertion mechanism with an injection needle (218) insertable into the skin of a patient by energy transmittable from the mechanical drive interface to the first mechanical interface if the first reservoir unit is attached to the drive unit,
the second reservoir unit comprising
a) a second housing (217'),
b) a second patch unit (201', 202') for attaching to a patient's body during administration,
c) a second counter engagement means (210') for releasable attachment of the drive unit to the second reservoir unit via the engagement means and the second counter engagement means and,
d) a second mechanical interface (214') connected to a second needle insertion mechanism with an injection needle (218') insertable into the skin of a patient by energy transmittable from the mechanical drive interface to the second mechanical interface if the second reservoir unit is attached to the drive unit;
**characterized in that** the shape of the first housing is different from the shape of the second housing.

2. The system according to claim 1, wherein the first reservoir unit (2) is adapted to receive a first medicament reservoir (3) of a first diameter and the second reservoir unit (2') is adapted to receive a second medicament reservoir (3') of a second diameter.

3. The system according to claim 1, wherein the patch unit (201, 202) for the first reservoir unit and the patch unit (201', 202') for the second reservoir unit are identical.

4. The system according to claim 1, wherein the first and the second needle insertion mechanisms are identical and are contained in a needle unit, affixed to the inside of the first and second reservoir unit.

5. The system according to claim 2, wherein the reusable drive unit (1) comprises an electric motor (111) for driving the plunger rod and for driving the mechanical drive interface (113).

6. The system according to claim 5, wherein the reusable drive unit (1) comprises a battery (110) for storing energy for the electric motor (111).

7. The system according to claim 1, wherein the drive unit is configured to dispense the entire amount of liquid drug from the reservoir (3, 3') continuously without interruption.

8. The system according to claim 1, wherein the drive unit is configured to dispense the entire amount of liquid drug in less than 60 minutes, preferably less than 45 minutes.

9. The system according to claim 1, wherein, when the first reservoir unit (2) is attached to the drive unit, the first patch unit (201, 202) is in a first rotational orientation relative to a longitudinal axis of the drive unit and wherein, when the second reservoir unit (2') is attached to the drive unit, the second patch unit (201', 202') is in a second rotational orientation relative to the longitudinal axis of the drive unit.

10. The system according to claim 6, wherein, when the first reservoir unit (2) is attached to the drive unit (1), a distance between a longitudinal center of the first reservoir (3) and the first patch unit (201, 202) is distinct from a distance between a longitudinal center of the second reservoir (3') and the second patch unit (201', 202'), when the second reservoir unit (2') is attached to the drive unit (1).

11. The system according to claim 6, wherein the first and the second engagement means are configured as a bayonet connection.

12. The system according to claim 6, wherein the first and the second engagement means are configured for a linear attachment of the drive unit to the reservoir unit, such that there is no relative rotation between the units.

13. The device according to any of the previous claims, wherein the possible reservoir volumes include at least a 10ml and a 20ml cartridge.
